# NEUE EUROPÄISCHE PATENTSCHRIFT

(11) **EP 1 662 234 B2**
(45) Veröffentlichungstag und Bekanntmachung des Hinweises auf die Entscheidung über den Einspruch: **19.11.2014**
(45) Hinweis auf die Patenterteilung: 05.01.2011
(21) Anmeldenummer: 05110736.5
(22) Anmeldetag: 15.11.2005
(51) Int. Cl.: G01D 9/00, A61L 2/28

(54) **Vorrichtung zur Erfassung und Speicherung von Meßwerten**
Measurement recording and storage device
Dispositif destiné à l'enregistrement et au stockage de données de mesure

(30) Priorität: 24.11.2004 DE 102004056796
(43) Veröffentlichungstag der Anmeldung: 31.05.2006
(73) Patentinhaber: ebro Electronic GmbH, 85055 Ingolstadt (DE)
(72) Erfinder: Klün, Wolfgang, 85049, Ingolstadt (DE)
(74) Vertreter: Schlief, Thomas P.

(56) Entgegenhaltungen:
- EP-A- 0 604 387
- EP-B- 0 671 956
- US-A- 6 156 267
- US-B1- 6 536 060
- US-B2- 6 532 794

## Beschreibung

Die vorliegende Erfindung betrifft eine Vorrichtung zur Erfassung, Sammlung und Speicherung von Meßwerten in einem temperatur- und/oder druck- und/oder feuchteabhängigen Prozeß. Die Vorrichtung weist mehrere Sensoren zum Aufnehmen von Messwerten auf. Die Sensoren sind hierbei im Inneren eines geschlossenen Prozessraumes angeordnet, welcher metallische Wandungen aufweist. Den Sensoren sind jeweils Mittel zum Abspeichern der Messwerte zugeordnet. Die Sensoren und die Mittel zur Abspeicherung der Messwerte sind zum Anordnen im Inneren des geschlossenen Prozessraumes ausgebildet. Weiterhin sind den Sensoren Mittel zum drahtlosen Übertragen von Messwerten an eine Empfangseinrichtung außerhalb des Prozessraumes zugeordnet. Insbesondere dient die Vorrichtung der Erfassung und Speicherung von Messwerten in einem Wasch- oder Sterilisationsprozess. Sie ist jedoch ebenso in einem Kühl- oder Erwärmungsprozess sowie in kombinierten Prozessen einsetzbar. Weiterhin betrifft die Erfindung ein Verfahren zur Überwachung eines temperatur- und/oder druck- und/oder feuchtigkeitsabhängigen Prozesses, insbesondere eines Sterilisationsprozesses, wobei eine Messwerterfassung durch mehrere Sensoren im Inneren eines geschlossenen Prozessraumes mit metallischen Wandungen erfolgt. Die erfassten Messwerte werden gesammelt und in mehreren, jeweils den Sensoren zugeordneten Datenloggern gespeichert. Die gespeicherten Messwerte werden mittels Funk an eine Empfangseinrichtung außerhalb des Prozessraumes übermittelt.

Wäschestücke, Operationswerkzeuge oder sonstige medizinische Materialien müssen vor der Wiederverwendung gereinigt und sterilisiert werden. Die Sterilisation erfolgt in der Regel durch Dampfsterilisation in Sterilisationskammern. Hierzu wird gesättigter Dampf in die Sterilisationskammer geleitet, welcher die Sterilisationsobjekte direkt berührt. Die Einhaltung gewisser Prozessparameter, insbesondere einer exakten Temperatur und eines gleichbleibenden Drucks, ist zur Sicherstellung einer korrekten Sterilisation zwingend erforderlich. Die Überwachung des Prozesses erfolgt durch entsprechende Vorrichtungen.

Die US 3,982,893 beschreibt eine derartige Überwachungseinrichtung. Sensoren zur kontinuierlichen Überwachung des Sterilisationsprozesses können hierbei direkt im Sterilisationsgut angeordnet werden. Anschließend wird in einem Analog-/Digitalwandler aus den Messwerten ein digitales Signal erzeugt, welches über Funk an eine in der Sterilisationskammer angeordnete Antenne gesendet wird. Über ein Kabel, welches durch die Gehäusewandungen der Sterilisationskammer hindurchgeführt wird, ist die Antenne mit weiteren Steuerungs- oder Kontrolleinrichtungen verbunden. Die Vorrichtung sieht hierbei eine ständige Überwachung und Übermittlung der Daten vor. Durch die Anordnung der Sensoren direkt im Sterilisationsgut und die Datenübermittlung über Funk ist es zwar möglich, die Temperatur im Inneren des Prozessgutes exakter zu erfassen, die Durchführung von Kabeln durch die Gehäusewandungen der Sterilisationskammer ist jedoch konstruktiv aufwendig. Darüber hinaus ist bei Störungen des Funkkontaktes keine Überwachung und Überprüfung des Sterilisationsprozesses mehr möglich.

Die EP 0 671 956 B1 sieht vor, eine Testeinheit in der Kammer eines Sterilisators anzuordnen. Die Bestandteile dieses austauschbaren Testmoduls können beispielsweise ein Temperatursensor und ein Drucksensor sein. Die Testeinheit wird an einer Stelle im Sterilisator nahe dem Ablauf angeordnet. Das Vorhandensein von Luft durch einen Temperatursensor kann hier besonders gut erfasst werden. Die durch die Testeinheit erfassten Daten werden über Funk an einen Empfänger außerhalb der Kammer zur Echtzeitüberwachung übertragen. Bei Störungen des Funkkontakts stehen keine Messdaten zur Verfügung; die Einhaltung der Prozessparameter kann somit nicht mehr überprüft werden. Alternativ können die Daten auch intern aufgezeichnet und erst nach Beendigung des Prozesses ausgelesen und ausgewertet werden. Störungen des laufenden Prozesses können hierbei jedoch nicht erkannt werden. Nachteilig bei dieser Anordnung ist weiterhin, dass Prozessdaten nur an einer einzigen Stelle des Sterilisators erfasst werden können. Darüber hinaus enthält die Testeinheit weitere Einrichtungen zur Datenverarbeitung und Signalerzeugung, so dass der Aufbau einer derartigen Testeinheit vergleichsweise aufwendig ist.

Aufgabe der vorliegenden Erfindung ist es, eine Vorrichtung zu schaffen, welche eine Auswertung von Messdaten sowohl während als auch nach Beendigung des Prozesses ermöglicht, und zugleich die konstruktive Ausführung derartiger Vorrichtungen zu vereinfachen.

Die Aufgabe wird gelöst mit einer Vorrichtung und einem Verfahren gemäß der unabhängigen Ansprüche.

Die erfindungsgemäße Vorrichtung zur Erfassung, Sammlung und Speicherung von Messwerten kann in Wasch-, Kühl- oder Erwärmungsprozessen und insbesondere in Sterilisationsprozessen verwendet werden. Die Vorrichtung umfasst mehrere Sensoren zum Aufnehmen von Messwerten, welche im Inneren eines geschlossenen Prozessraumes mit metallischen Wandungen angeordnet sind. Üblicherweise sind die Wandungen derartiger Prozessräume aus Metall ausgebildet, da z.T. sehr hohe oder sehr tiefe Temperaturen und hohe Drücke auftreten. Die Vorrichtung enthält zudem mehrere, jeweils den Sensoren zugeordnete Mittel zur Abspeicherung der Messwerte, mehrere Datenlogger zum Abspeichern der Messwerte in zeitlicher Abfolge, in vorgegebenen Zeitabständen. Die Datenlogger speichern beispielsweise alle 10 Sekunden den jeweilig aktuellen Messwert des zugeordneten Sensors. Den Sensoren sind jeweils Mittel zur drahtlosen Übertragung der abgespeicherten Messwerte an eine außerhalb des geschlossenen Prozessraumes angeordnete Empfangseinrichtung vorgesehen. Erfolgt die Übertragung der gespeicherten Messwerte aus dem Inneren des Prozessraumes an eine außerhalb angeordnete Empfangseinrichtung, entfallen die konstruktiv aufwendigen Kabeldurchführungen, so dass der Bau von entsprechenden Geräten erheblich vereinfacht ist. Die Verwendung entsprechender Übertragungsmittel ermöglicht hierbei die Übertragung von Messdaten aus einem nahezu vollständig von Metallwandungen umgebenen geschlossenen Prozessraum.

Erfindungsgemäß sind die Mittel zur drahtlosen Übertragung der abgespeicherten Messwerte zum intermittierenden drahtlosen Übertragen von abgespeicherten Messwerten aus den verschiedenen Datenloggern in vorgegebenen oder vorgebbaren Zeitabständen ausgebildet. Die empfangende Einrichtung außerhalb des Prozessraumes kann beispielsweise nacheinander Messwerte von den verschiedenen Datenloggern abrufen.

Die mehreren Sensoren und die diesen zugeordneten Datenlogger, insbesondere mehrere Sensor-Datenlogger-Einheiten, sind vorzugsweise an verschiedenen Orten des Prozessgutes und/oder des Prozessraumes angeordnet. Örtliche Schwankungen der Prozessbedingungen können durch eine derartige Anordnung besonders zuverlässig erfasst werden.

Vorzugsweise speichern die Datenlogger die von den jeweiligen Sensoren aufgenommen Messwerte in vorgegebenen Abständen, beispielsweise alle 10 Sekunden. Die abrufende Empfangseinrichtung ruft dann beispielsweise jede Minute die inzwischen sechs neuen Messwerte aus diesem Datenlogger ab. Anschließend können die entsprechenden sechs Messwerte eines anderen Datenloggers an anderer Stelle des Prozessraumes abgerufen werden.

Vorzugsweise enthält die Vorrichtung wenigstens einen Sensor zur Erfassung der Temperatur in dem Prozessraum. Durch einen Temperatursensor kann beispielsweise in einem Sterilisationsprozess das Vorhandensein schädlicher Luft besonders gut nachgewiesen werden. Insbesondere, wenn mehrere Sensoren zur Erfassung der Temperatur angeordnet sind, kann der Prozess besonders gut überwacht werden und eine für den Prozess erforderliche Temperaturverteilung sichergestellt werden.

Weiterhin ist es vorteilhaft, wenn die Vorrichtung einen Sensor zur Erfassung des Drucks in dem Prozessraum beinhaltet, so dass die Überwachung der maßgebenden Prozessparameter verbessert ist.

Besonders bevorzugt ist jeweils ein Sensor mit einem Datenlogger gekoppelt. Sensor und Datenlogger können hierbei in einem gemeinsamen Gehäuse oder getrennt - verbunden mit einer Datenleitung - angeordnet sein. Die Daten bzw. Prozessparameter aus dem oder den Datenloggern können daher während der Prozessüberwachung, vorzugsweise durch drahtlose Übertragung in zeitlichen Abständen, und zusätzlich auch am Ende des Prozesses nach Entnahme der Datenlogger aus dem Prozessraum überprüft werden.

Besonders exakte Messwerte können erreicht werden, wenn die Sensoren direkt im Prozessgut anordenbar sind. Hierdurch kann sichergestellt werden, dass die notwendigen Temperaturen auch im Inneren des Prozessgutes erreicht werden. Im Falle eines Sterilisationsprozesses kann somit sichergestellt werden, dass die Temperatur durch Kondensation entstanden ist und somit eine hinreichende Sterilisation stattgefunden hat. Ebenso kann der mindestens eine Sensor in einem Garprozess oder einem Kühlprozess vorteilhaft zur Messung der Kerntemperatur des Prozessgutes eingesetzt werden.

Beinhalten die Mittel zur drahtlosen Übertragung der Messwerte aus dem geschlossenen Prozessraum eine Funkeinrichtung, ist eine zuverlässige Datenübertragung an die außerhalb angeordnete Empfangseinrichtung möglich. Die Empfangseinrichtung der drahtlosen Übertragungseinrichtung ist vorzugsweise derart ausgebildet, dass sie an einer Außenwand des geschlossenen Prozessraumes angeordnet werden kann. Besonders vorteilhaft ist es hierbei, wenn die Empfangseinrichtung an der Außenwand lösbar, beispielsweise mittels einer Magnethalterung oder einer Clipseinrichtung, angeordnet ist. Die Empfangseinrichtung ist hierdurch besonders leicht zuordenbar, auffindbar und austauschbar. Auch können Wartungsarbeiten auf einfache Weise durchgeführt werden.

Eine konstruktiv einfache und kostengünstige Ausführung der Erfindung ergibt sich dadurch, dass eine Sende- und/oder Empfangsantenne der Funkeinrichtung durch eine Spule gebildet ist.

Eine besonders vorteilhafte Weiterbildung der Erfindung sieht vor, dass der Vorrichtung eine Einrichtung zur Verarbeitung und/oder Ausgabe der Messwerte außerhalb des geschlossenen Prozessraumes zugeordnet ist. Dies ermöglicht das Anzeigen der Prozessparameter und hierdurch auch ein Eingreifen in den Prozess durch das Bedienpersonal. Ebenso ist es möglich, die Messwerte weiterzuverarbeiten und den Prozess mittels der Messwerte zu steuern. Weiterhin ist es möglich, den Verlauf des Prozesses anhand der ausgegebenen Messwerte nachträglich zu überprüfen.

Bei einer weiteren vorteilhaften Ausführungsform der Erfindung ist die Empfangseinrichtung mittels einer Kabelverbindung oder sonstigen Datenverbindung (z.B. IR-Übertragung) mit einer Verarbeitungs- und/oder Ausgabeeinheit außerhalb des Prozessraumes verbunden. Insbesondere ist es hierbei konstruktiv einfach, eine feste Datenverbindung zwischen Empfangseinrichtung und Verarbeitungs- und/oder Ausgabeeinheit vorzusehen. Auch kann vorgesehen sein, dass die einzelnen Datenlogger nach Entnahme aus dem Prozessraum direkt mit einer Verarbeitungs- und/oder Ausgabeeinheit (ohne Zwischenschaltung der Empfangseinrichtung) verbunden werden können, um die Prozessparameter auszulesen.

In dem erfindungsgemäßen Verfahren zur Überwachung eines temperatur- und/oder druck- und/oder feuchtigkeitsabhängigen Prozesses, insbesondere eines Sterilisationsprozesses, erfolgt eine Messwerterfassung durch mehrere Sensoren im Inneren eines geschlossenen Prozessraumes mit metallischen Wandungen. Die Messwerte werden gesammelt, in mehreren den Sensoren zugeordneten Datenloggern gespeichert und drahtlos an eine Empfangseinrichtung außerhalb des Prozessraumes übermittelt. Erfindungsgemäß werden die gespeicherten Messwerte während des Prozesses in vorgegebenen oder vorgebbaren Zeitabständen jeweils zeitlich versetzt drahtlos an die Empfangseinrichtung übermittelt. Dies ermöglicht sowohl eine Online-Überwachung als auch eine nachträgliche Beurteilung der Prozessparameter. Kurzfristige Unterbrechungen der Übertragung wirken sich wegen der Speicherung der Messwerte nicht negativ aus.

Das erfindungsgemäße Verfahren ermöglicht darüber hinaus vorteilhafterweise, die maßgebenden Parameter durch mehrere Sensoren an verschiedenen Stellen zu erfassen und in entsprechenden Datenloggern abzuspeichern. Die Übermittlung der Daten verschiedener Datenlogger an die Empfangseinrichtung kann dann jeweils zeitversetzt erfolgen, so dass mehrere Prozessparameter zuverlässig erfasst werden können und die Empfangseinrichtung dennoch konstruktiv einfach gehalten werden kann. Eine Verarbeitung der Daten vor der Übermittlung ist hierdurch nicht nötig. Die Datenübertragung an die Empfangseinrichtung erfolgt durch Funkwellen.

Eine vorteilhafte Ausführung der Erfindung sieht vor, dass die maßgebenden Parameter direkt in dem Prozessgut erfasst werden. Die Qualität des Prozesses kann hierdurch besonders zuverlässig überwacht werden.

Eine weitere vorteilhafte Weiterbildung des Verfahrens sieht vor, dass die Meßwerte von der Empfangseinrichtung an eine Verarbeitungs- und/oder Ausgabeeinheit zur Anzeige der Messwerte und/oder zur Steuerung des Prozesses geleitet werden. Der Prozess kann hierdurch vollautomatisch gesteuert werden, oder durch das Eingreifen von Bedienpersonal. Ebenso besteht auch die Möglichkeit, die Messwerte nach Prozessablauf zu überprüfen.

Weitere Vorteile der Erfindung werden anhand der nachfolgenden Ausführungsbeispiele näher erläutert. Es zeigen
- **Fig. 1**: eine schematische Darstellung einer erfindungsgemäßen Vor- richtung mit einer nachgeordneten Auswerte- und Anzeigeein- heit;
- **Fig. 2**: eine schematische Detailansicht eines Datenloggers mit inter- nem Sensor, und
- **Fig. 3**: eine schematische Detailansicht eines Datenloggers mit exter- nem Sensor.

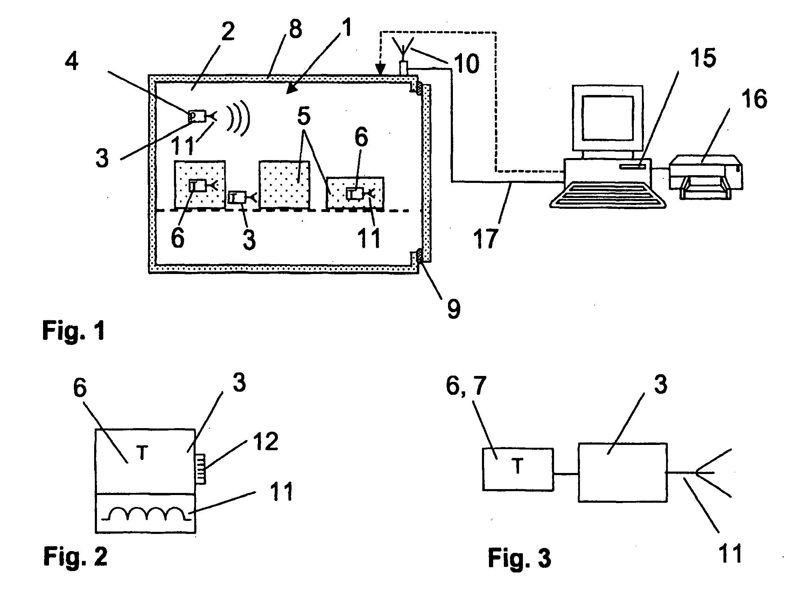

Figur 1 zeigt eine schematische Darstellung einer erfindungsgemäßen Vorrichtung 1, welche in einem temperatur-, druck- und/oder feuchtigkeitsabhängigen Prozess eingesetzt werden kann. Der Prozess findet hierbei in einem geschlossenen Prozessraum 2 wie beispielsweise einem Kühlschrank, Ofen, Wasch- oder Spülgerät, einem Autoklaven oder einem Dampfsterilisator statt. Ebenso ist der Einsatz in Reinigungs- und Desinfektionsautomaten möglich. Die Vorrichtung 1 besteht aus mehreren Sensoren 4, 6 und jeweils zugeordnetem Datenlogger 3 zur Erfassung und Speicherung von Messwerten sowie aus mehreren drahtlosen Übertragungseinrichtungen 11 und einer Empfangseinrichtung 10, wobei diese beiden Einrichtungen vorliegend als Antennen 10, 11 ausgebildet sind. Auf diese Weise können die Messwerte aus den Datenloggern 3 zur außerhalb des Prozessraums 2 angeordneten und als Empfangseinrichtung wirkenden Antenne 10 übermittelt werden. Vorliegend sind die Datenlogger 3 mit internen Sensoren 4, 6 für Druck und Temperatur verbunden, wobei der Begriff "intern" bedeutet, dass Logger und Sensor in einem gemeinsamen Gehäuse untergebracht sind. Ebenso ist jedoch der Einsatz von Loggern 3 mit externen Sensoren 7, wie in Fig. 3 dargestellt, oder auch von Loggern 3, welche zwei Sensoren oder einen kombinierten Druck-Temperatur-Sensor aufweisen, möglich.

Die Messwerte werden in den Datenloggern 3 gesammelt und gespeichert, vorzugsweise in zeitlichen Abständen, beispielsweise alle 10, 20 oder 30 Sekunden der jeweils aktuelle Messwert. Die Messgrößen können hierbei besonders exakt durch eine Plazierung der Logger 3 direkt im Prozessgut 5 erfasst werden. Die Überwachung bzw. Überprüfung des Prozesses ist hierdurch besonders einfach möglich, da die maßgebenden Parameter sowohl an mehreren Stellen im Prozessgut 5 wie auch im Prozessraum 2 ermittelt und verglichen werden können.

Die gespeicherten Messwerte werden anschließend drahtlos an die Empfangseinrichtung 10 übermittelt, welche außerhalb des Prozessraumes 2 und an diesem Prozessraum 2 insbesondere mittels einer nicht näher dargestellten Halteeinrichtung (beispielsweise Magnet- oder Clipsverbindung) angeordnet ist. Durch diese Anordnung der Empfangseinrichtung 10 sind aufwendige Kabeldurchführungen durch die Wandungen des Prozessraumes 2 nicht erforderlich.

Weiterhin ist erfindungsgemäß vorgesehen, dass die gespeicherten Messwerte in vorgegebenen oder vorgebbaren Zeitabständen an die Empfangseinrichtung 10 übermittelt werden. Die Messdaten sind hierdurch direkt online verfügbar; gelegentliche Störungen der Übertragung wie bei einer Echtzeitüberwachung wirken sich jedoch nicht negativ aus, so dass die Überwachung des Prozesses zuverlässig durchgeführt werden kann. Darüber hinaus ermöglicht die intermittierende Übertragung, die Messwerte verschiedener Logger 3 an eine einzige Empfangseinrichtung 10 zu übertragen, da die Übertragung zeitlich versetzt erfolgen kann, insbesondere durch Abrufen der zwischenzeitlich neu abgespeicherten Messwerte in den verschiedenen Loggern 3. Durch die Speicherung der Messwerte in den Loggern 3 ist jedoch auch eine nachträgliche Überprüfung des Prozesses möglich.

Die Datenlogger 3 sowie die Mittel zur drahtlosen Übertragung 11 der Messwerte werden in dem geschlossenen Prozessraum 2 angeordnet. Bestehen die Wandungen 8 des Prozessraumes 2 aus Metallen, kann die Signalübertragung trotz der somit resultierenden Faraday-Abschirmung beispielsweise durch die Gummidichtungen 9 erfolgen, wie Versuche überraschenderweise ergeben haben. Die Datenlogger 3 müssen hierzu über eine entsprechend hohe Sendeleistung verfügen.

Die Empfangseinrichtung 10 ist im dargestellten Beispiel über eine feste Datenverbindung 17 an einen Rechner 15 zur Verarbeitung und Ausgabe der Messdaten angeschlossen. Die Verarbeitung in dem Rechner 15 erfolgt durch eine entsprechende Software, ein Ausdruck von Prozess-Protokollen ist über den Drucker 16 möglich. Die Rechnereinheit 15 kann zusätzlich mit einer Steuerungseinrichtung zur Einstellung der Prozessparameter versehen sein, wie durch die gestrichelte Linie angedeutet.

Fig. 2 zeigt eine Detailansicht eines Temperaturloggers 3, wobei als Sendeantenne 11 eine Spule an dem Logger 3 angeordnet ist. Der Logger 3 verfügt zusätzlich über Mittel 12 zum Anschluss an ein Interface, welches ein Auslesen der Daten auch nach Entnahme der Logger 3 aus dem Prozessraum 2 ermöglicht. Dies erlaubt eine Aussage über die Qualität des abgelaufenen Prozesses, auch wenn eine Online-Überwachung beispielsweise wegen Störungen der Übertragung nicht möglich ist.

Eine erfindungsgemäße Vorrichtung 1 ist besonders vorteilhaft in einem Sterilisationsprozess in einem Dampfsterilisator einsetzbar. Für die Überprüfung und Überwachung eines Sterilisationsprozesses sind Datenlogger 3 mit Temperatur- und Drucksensoren 4, 6 einzusetzen. Die Logger 3 müssen hierbei wasser- und druckdicht ausgeführt sein. Für die Erfassung des Temperatur- und Druckverlaufs einer Chargenladung, beispielsweise medizinischer Instrumente, kann es ausreichend sein, einen Datenlogger 3 mit Temperaturfühler 6 und Drucksensor 4 zwischen den Sterilisiergütern 5 zu plazieren. Häufig ist jedoch die Erfassung der Temperaturwerte an verschiedenen Stellen des Prozessraumes 2 bzw. des Prozessgutes 5 notwendig, so dass bis zu 12 Temperaturlogger und ein Drucklogger in der Sterilisationskammer plaziert werden. Um auch die Temperatur in kleineren Hohlräumen des Sterilisiergutes 5, welche im Sterilisationsprozess erhebliche Probleme bereiten, exakt erfassen zu können, ist der Einsatz von Datenloggern 3 mit externen Sensoren 7, wie in Fig. 3 dargestellt, sinnvoll. Dieser eignet sich besonders für Temperaturmessungen in Hohlräumen, in welche der Sensor 7 des Loggers 3 eingeführt werden kann.

Die Vorrichtung 1 kann jedoch auch in jedem anderen Prozess mit Temperatur, Druck- und Feuchteparametern eingesetzt werden. Eine Anpassung an die jeweilige Aufgabe kann durch die Auswahl der Datenlogger 3 sowie durch die Programmierung des Messtaktes erfolgen. Bei einem Einsatz der erfindungsgemäßen Vorrichtung 1 in Kühl- oder Garprozessen ist es besonders vorteilhaft, dass die Kerntemperatur des Prozessgutes 5 erfasst werden kann.

Die Erfindung ist nicht auf die dargestellten Ausführungsbeispiele beschränkt. Abwandlungen und Kombinationen im Rahmen der Patentansprüche fallen ebenfalls unter die Erfindung.

## Patentansprüche

1. Vorrichtung (1) zur Erfassung, Sammlung und Speicherung von Messwerten in einem temperatur- und/oder druck- und/oder feuchtigkeitsabhängigen Prozess im Inneren eines geschlossenen Prozessraumes (2), welcher metallische Wandungen aufweist, insbesondere in einem Sterilisationsprozess, mit mehreren Sensoren (4, 6) zum Aufnehmen von Messwerten, mit mehreren jeweils den Sensoren zugeordneten Mitteln zur Abspeicherung der Messwerte, wobei die Sensoren (4, 6) und die Mittel zur Abspeicherung der Messwerte zum Anordnen im Inneren eines geschlossenen Prozessraumes (2) ausgebildet sind, mit einer Empfangseinrichtung (10), welche zum Anordnen außerhalb des Prozessraumes (2) ausgebildet ist, sowie mit den Sensoren zugeordneten Mitteln (11) zur drahtlosen Übertragung von Messwerten an die Empfangseinrichtung (10),
**dadurch gekennzeichnet,**
- **dass** die mehreren Sensoren zum derartigen Platzieren an unterschiedlichen Stellen des Prozessgutes und/oder des Prozessraumes ausgebildet sind, dass die örtlichen Schwankungen der Prozessbedingungen an diesen unterschiedlichen Stellen erfassbar sind,
- **dass** die Mittel zur Abspeicherung der Messwerte als mehrere Datenlogger (3) ausgebildet sind,
- **dass** die Mittel (11) zur drahtlosen Übertragung zum intermittierenden drahtlosen Übertragen von in den Datenloggern (3) abgespeicherten Messwerten in vorgegebenen oder vorgebbaren Zeitabständen ausgebildet sind, und
- **dass** dle Empfangseinrichtung (10) zum Empfang der abgespeicherten Messwerte von mehreren Sensoren (4, 6) so ausgebildet ist, dass die Übertragung der in den mehreren Datenloggern (3) zur Abspeicherung der Messwerte gespeicherten Messwerte an die eine Empfangseinrichtung (10) zeitlich versetzt erfolgt.

2. Vorrichtung nach Anspruch 1, **dadurch gekennzeichnet, dass** die Vorrichtung (1) mindestens einen Sensor (6) zur Erfassung der Temperatur in dem Prozessraum (2) beinhaltet.

3. Vorrichtung nach einem der vorhergehenden Ansprüche, **dadurch gekennzeichnet, dass** die Vorrichtung (1) mindestens einen Sensor (4) zur Erfassung des Drucks in dem Prozessraum (2) beinhaltet.

4. Vorrichtung nach einem der vorhergehenden Ansprüche, **dadurch gekennzeichnet, dass** die Sensoren (4, 6) direkt im Prozessgut (5) anordenbar sind.

5. Vorrichtung nach einem der vorhergehenden Ansprüche, **dadurch gekennzeichnet, dass** die Mittel (11) zur drahtlosen Übertragung der Messwerte aus dem geschlossenen Prozessraum (2) eine Funkeinrichtung beinhalten.

6. Vorrichtung nach einem der vorhergehenden Ansprüche, **dadurch gekennzeichnet, dass** die Empfangseinrichtung (10) zum Abrufen der Messwerte aus den Mitteln zur Abspeicherung der Messwerte ausgebildet ist.

7. Vorrichtung nach einem der vorhergehenden Ansprüche, **dadurch gekennzeichnet, dass** eine Sendeantenne der Funkeinrichtung und/oder eine Empfangsantenne der Empfangseinrichtung (10) durch eine Spule gebildet ist.

8. Vorrichtung nach einem der vorhergehenden Ansprüche, **dadurch gekennzeichnet, dass** die Empfangseinrichtung (10) an einer Außenwand des geschlossenen Prozessraumes (2) angeordnet ist.

9. Vorrichtung nach einem der vorhergehenden Ansprüche, **dadurch gekennzeichnet, dass** die Empfangseinrichtung (10) an der Außenwand des geschlossenen Prozessraumes (2) lösbar, beispielsweise mit einer Magnethalterung oder einer Clipsvorrichtung, befestigbar ist.

10. Vorrichtung nach einem der vorhergehenden Ansprüche, **dadurch gekennzeichnet, dass** der Vorrichtung (1) eine Einrichtung zur Verarbeitung und/oder Ausgabe der Messwerte (15, 16) außerhalb des geschlossenen Prozessraumes (2) zugeordnet ist.

11. Vorrichtung nach einem der vorhergehenden Ansprüche, **dadurch gekennzeichnet, dass** die Empfangseinrichtung (10) Mittel (17) zur Übertragung der Messwerte an eine Verarbeitungs- und/oder Ausgabeeinheit (15, 16) außerhalb des Prozessraumes (2) durch eine feste Datenverbindung enthält.

12. Vorrichtung nach einem der vorhergehenden Ansprüche, **dadurch gekennzeichnet, dass** die Mittel zur Abspeicherung der Messwerte Mittel (12) zur Übertragung der Messwerte an eine Verarbeitungs- und/oder Anzeigeeinheit (15, 16) außerhalb des Prozessraumes (2) durch eine feste Datenverbindung enthalten.

13. Verfahren zur Überwachung eines temperatur- und/oder druckund/oder feuchtigkeitsabhängigen Prozesses im Inneren eines geschlossenen Prozessraumes (2), welcher metallische Wandungen aufweist, insbesondere eines Sterilisationsprozesses, wobei eine Messwerterfassung durch mehrere in dem Prozessgut und/oder dem Prozessraum (2) angeordnete Sensoren (4, 6) erfolgt, die erfassten Messwerte gesammelt und in mehreren, jeweils den Sensoren (4, 6) zugeordneten und im Inneren des Prozessraumes (2) angeordneten Mitteln zur Abspeicherung der Messwerte gespeichert werden, und die gespeicherten Messwerte drahtlos an eine Empfangseinrichtung (10) außerhalb des Prozessraumes (2) übermittelt werden,
**dadurch gekennzeichnet,**
- **dass** die mehreren Sensoren (4, 6) zur Erfassung der maßgebenden Parameter an unterschiedlichen Stellen des Prozessguts (5) und/oder des Prozessraumes (2) derart platziert werden, dass örtliche Schwankungen der Prozessbedingungen an diesen unterschiedlichen Stellen erfassbar sind,
- **dass** die Mittel zur Abspeicherung der Messwerte als mehrere Datenloggor (3) ausgebildet sind, und
- **dass** die in den mehreren Datenlaggern (3) gespeicherten Messwerte während des Prozesses intermittierend in vorgegebenen oder vorgebbaren Zeitabständen und jeweils zeitlich versetzt an die Empfangseinrichtung (10) übertragen werden.

14. Verfahren nach dem vorherigen Anspruch, **dadurch gekennzeichnet, dass** die Empfangseinrichtung (10) die Messwerte aus den Datenloggern (3) abruft.

15. Verfahren nach einem der vorherigen Ansprüche, **dadurch gekennzeichnet, dass** die gespeicherten Messwerte mittels Funk mit einer derart hohen Sendeleistung übertragen werden, dass die Funkwellen durch Dichtungen des Prozeßraumes (2) zur Empfangseinrichtung (10) gefunkt werden.

16. Verfahren nach einem der vorherigen Ansprüche, **dadurch gekennzeichnet, dass** die Erfassung der maßgebenden Parameter direkt in dem Prozessgut (5) erfolgt.

17. Verfahren nach einem der vorherigen Ansprüche, **dadurch gekennzeichnet, dass** die Messwerte von der Empfangseinrichtung (10) an eine Verarbeitungs- und/oder Ausgabeeinheit (15, 16) zur Anzeige der Messwerte und/oder zur Steuerung des Prozesses geleitet werden.

## Claims

1. Device (1) for acquiring, compiling and storing measured values in a temperature and/or pressure and/or moisture-dependent process inside a closed process chamber (2) with metallic walls, especially in a sterilization process, with multiple sensors (4, 6) for acquiring measured values, with multiple means respectively assigned to the sensors for storing the measured values, wherein the sensors (4, 6) and the means for storing the measured values are designed for arrangement inside a closed process chamber (2), with a receiver unit (10) being designed for arrangement outside the process chamber (2), and with means (11) assigned to the sensors for wireless transmission of measured values to the receiver unit (10)
**characterized in**
- **that** the plurality of sensors are designed to be placed at different positions of the process material and/or the process chamber, such that the local variations of the process conditions at said different positions can be captured,
- **that** the means for saving the measured values are implemented as a plurality of dataloggers (3),
- **that** the means (11) for wireless transmission are designed for the wireless intermittent transmission of measured values stored in the dataloggers (3) at preset or settable time intervals, and
- **that** the receiver unit (10) for the reception of the stored measured values of multiple sensors (4, 6) is designed such that the transmission of the measured values, that are stored in the multiple dataloggers (3) for storing the measured values, to the receiver unit (10) is carried out delayed.

2. Device according to claim 1, **characterized in that** the device (1) comprises at least one sensor (6) for acquiring the temperature inside the process chamber (2).

3. Device according to one of the preceding claims, **characterized in that** the device (1) comprises at least one sensor (4) for acquiring the pressure inside the process chamber (2).

4. Device according to one of the preceding claims, **characterized in that** the sensors (4, 6) can be positioned directly in the process material (5).

5. Device according to one of the preceding claims, **characterized in that** the means (11) for wireless transmission of the measured values from the closed process chamber (2) comprise a radio device.

6. Device according to one of the preceding claims, **characterized in that** the receiver unit (10) is designed to retrieve the measured values from the means for storing the measured values.

7. Device according to one of the preceding claims, **characterized in that** a transmitting antenna for the radio system and/or a receiving antenna for the receiver unit (10) is formed by a coil.

8. Device according to one of the preceding claims, **characterized in that** the receiver unit (10) is positioned on an outer wall of the closed process chamber (2).

9. Device according to one of the preceding claims, **characterized in that** the receiver unit (10) can be mounted separably on the outer wall of the closed process chamber (2), for example via a magnetic mount or a clip device.

10. Device according to one of the preceding claims, **characterized in that** a device for processing and/or displaying the measured values (15, 16) is allocated to the device (1) outside of the closed process chamber (2).

11. Device according to one of the preceding claims, **characterized in that** the receiver unit (10) contains means (17) for transmitting the measured values to a processing and/or display unit (15, 16) outside the process chamber (2) via a permanent data connection.

12. Device according to one of the preceding claims, **characterized in that** the means (3) for storing the measured values comprise means (12) for transmitting the measured values to a processing and/or display unit (15, 16) outside the process chamber (2) via a permanent data connection.

13. Process for monitoring a temperature and/or pressure and/or moisture-dependent process inside a closed process chamber (2) with metallic walls, especially a sterilization process, wherein an acquisition of measured values is accomplished by means of multiple sensors (4, 6) positioned inside the process material and/or the process chamber (2), the acquired measured values are compiled and stored in multiple means for saving the measured values respectively assigned to the sensors (4, 6) and positioned inside the process chamber (2), and the stored measured values are transmitted wirelessly to a receiver unit (10) located outside of the process chamber (2), **characterized in**
- **that** the plurality of sensors (4, 6) for acquisition of the primary parameters are positioned at various locations of the process material (5) and/or the process chamber (2) such that local variations in the process conditions at said different locations can be captured,
- **that** the means for saving the measured values are implemented as a plurality of dataloggers (3), and
- **that** the measured values stored in multiple data loggers (3) are intermittently transmitted during the process at preset or settable time intervals and respectively with a time shift to the receiver unit (10).

14. Process according to one of the preceding claims, **characterized in that** the receiver unit (10) retrieves the measured values from the data loggers (3).

15. Process according to one of the preceding claims, **characterized in that** the stored measured values are transmitted via radio at such a strong transmitting power that the waves are radioed to the receiver unit (10) through seals of the process chamber (2).

16. Process according to one of the preceding claims, **characterized in that** the primary parameters are acquired directly within the process material (5).

17. Process according to one of the preceding claims, **characterized in that** the measured values are passed on from the receiver unit (10) to a processing and/or display unit (15, 16) for the display of the measured values and/or for controlling the process.

## Revendications

1. Dispositif (1) pour saisir, collecter et mémoriser des valeurs de mesure dans un processus dépendant de la température et/ou de la pression et/ou de l'humidité à l'intérieur d'une chambre de processus fermée (2) comportant des parois métalliques, en particulier dans un processus de stérilisation, avec plusieurs capteur (4, 6) pour saisir des valeurs de mesure, avec plusieurs moyens attribués respectivement auxdits capteurs pour mémoriser lesdites valeurs de mesure, sachant que les capteurs (4, 6) et les moyens pour mémoriser les valeurs de mesure sont adaptés pour être disposés à l'intérieur d'une chambre de processus fermée (2), avec un dispositif de réception (10) adapté pour être disposé hors de la chambre de processus (2), et avec des moyens (11) attribués auxdits capteurs pour la transmission sans fil de valeurs de mesure au dispositif de réception (10), **caractérisé en ce que**
- les plusieurs capteurs pour une telle disposition à divers endroits de la matière en traitement et/ou de la chambre de processus se présentent sous une forme telle que les fluctuations locales des conditions de traitement à ces différents endroits puissent être saisies,
- que les moyens pour la mémorisation des valeurs de mesure se présentent sous la forme de plusieurs enregistreurs de données (3),
- que les moyens (11) pour la transmission sans fil sont adaptés pour la transmission intermittente de valeurs de mesure mémorisées dans les enregistreurs de données (3) à des intervalles de temps alloués ou allouables, et
- que le dispositif de réception (10) pour la réception des valeurs de mesure mémorisées depuis plusieurs capteurs (4, 6) se présente sous une forme telle que la transmission, au dispositif de réception (10), des valeurs de mesure mémorisées dans les multiples enregistreurs de données (3) pour la mémorisation des valeurs de mesure, soit effectuée en temps différé.

2. Dispositif selon la revendication 1, **caractérisé en ce que** le dispositif (1) comporte au moins un capteur (6) pour la saisie de la température dans la chambre de processus (2).

3. Dispositif selon l'une des revendications précédentes, **caractérisé en ce que** le dispositif (1) comporte au moins un capteur (4) pour la saisie de la pression dans la chambre de processus (2).

4. Dispositif selon l'une des revendications précédentes, **caractérisé en ce que** les capteurs (4, 6) peuvent être disposés directement dans la matière en traitement (5).

5. Dispositif selon l'une des revendications précédentes, **caractérisé en ce que** les moyens (11) pour la transmission sans fil des valeurs de mesure depuis la chambre de processus fermée (2) comportent un dispositif radioélectrique.

6. Dispositif selon l'une des revendications précédentes, **caractérisé en ce que** le dispositif de réception (10) est adapté pour extraire les valeurs de mesure depuis les moyens pour la mémorisation des valeurs de mesure.

7. Dispositif selon l'une des revendications précédentes, caractérisé en ce q'une antenne d'émission du dispositif radioélectrique et/ou une antenne de réception du dispositif de réception (10) sont/est constituée(s) par une bobine.

8. Dispositif selon l'une des revendications précédentes, **caractérisé en ce que** le dispositif de réception (10) est disposé à une paroi extérieure de la chambre de processus fermée (2).

9. Dispositif selon l'une des revendications précédentes, **caractérisé en ce que** le dispositif de réception (10) est fixé de manière détachable à une paroi extérieure de la chambre de processus fermée (2), par exemple au moyen d'une attache magnétique ou d'un dispositif à clip.

10. Dispositif selon l'une des revendications précédentes, **caractérisé en ce qu'**un dispositif pour le traitement et/ou la sortie des valeurs de mesure (15, 16) en dehors de la chambre de processus fermée (2) est attribué au dispositif (1).

11. Dispositif selon l'une des revendications précédentes, **caractérisé en ce que** le dispositif de réception (10) comporte des moyens (17) pour la transmission des valeurs de mesure à une unité de traitement et/ou de sortie (15, 16) en dehors de la chambre de processus (2) via une connexion de données fixe.

12. Dispositif selon l'une des revendications précédentes, **caractérisé en ce que** les moyens pour la mémorisation des valeurs de mesure (comporte des moyens (12) pour la transmission des valeurs de mesure à une unité de traitement et/ou de sortie (15, 16) en dehors de la chambre de processus (2) via une connexion de données fixe.

13. Procédé pour surveiller un processus dépendant de la température et/ou de la pression et/ou de l'humidité à l'intérieur d'une chambre de processus fermée(2) comportant des parois métalliques, en particulier un processus de stérilisation, sachant qu'une acquisition de valeurs de mesure est réalisée par plusieurs capteurs (4, 6) disposés dans la matière en traitement et/ou la chambre de processus (2), les valeurs de mesure saisies sont collectées et mémorisées dans plusieurs moyens pour la mémorisation des valeurs de mesure attribués respectivement aux capteurs (4, 6) et disposés à l'intérieur de la chambre de processus (2), et les valeurs de mesure mémorisées sont transmises sans fil à un dispositif de réception (10) en dehors de la chambre de processus (2), **caractérisé en ce que**,
- les plusieurs capteurs (4, 6) pour la saisie des paramètres déterminants sont disposés à divers endroits dans la matière en traitement (5) et/ou de la chambre de processus (2) de sorte que des fluctuations locales des conditions de traitement à ces différents endroits puissent être saisies,
- que les moyens pour la mémorisation des valeurs de mesure se présentent sous la forme de plusieurs enregistreurs de données (3) et
- que les valeurs de mesure mémorisées dans les multiples enregistreurs de données (3) sont transmises de manière intermittente au dispositif de réception (10) durant le processus, à des intervalles de temps alloués ou allouables et respectivement en temps différé.

14. Procédé selon la revendication précédente, **caractérisé en ce que** le dispositif de réception (10) extrait les valeurs de mesure depuis les enregistreurs de données (3).

15. Procédé selon l'une des revendications précédentes, **caractérisé en ce que** les valeurs de mesure mémorisées sont transmises par ondes radioélectriques à une puissance d'émission aussi élevée que les ondes radioélectriques sont émises vers le dispositif de réception (10) à travers les joints de la chambre de processus (2).

16. Procédé selon l'une des revendications précédentes, **caractérisé en ce que** l'acquisition des paramètres déterminants s'effectue directement dans la matière en traitement (5).

17. Procédé selon l'une des revendications précédentes, **caractérisé en ce que** les valeurs de mesure sont transmises depuis le dispositif de réception (10) a une unité de traitement et/ou de sortie (15, 16) en vue de l'affichage des valeurs de mesure et/ou du pilotage du processus.
